Europäisches Patentamt

European Patent Office

Office européen des brevets

(19)

(11) Numéro de publication : **0 062 565**
**B1**

(12) FASCICULE DE BREVET EUROPÉEN

(45) Date de publication du fascicule du brevet :
18.01.84

(51) Int. Cl.³ : **C 07 H 13/06**

(21) Numéro de dépôt : 82400535.9

(22) Date de dépôt : 24.03.82

(54) Procédé de fabrication d'esters de sucre et notamment de saccharose.

(30) Priorité : 06.04.81 FR 8106839

(43) Date de publication de la demande :
13.10.82 Bulletin 82/41

(45) Mention de la délivrance du brevet :
18.01.84 Bulletin 84/03

(84) Etats contractants désignés :
**BE CH DE FR GB LI NL**

(56) Documents cités :
**FR-A- 2 205 504**

(73) Titulaire : **BLOHORN S.A.**
**01 B.P. 1751**
**Abidjan 01 (CI)**

(72) Inventeur : **Musso, Sandro**
**B.P. 1751**
**Abidjan (CI)**
Inventeur : **Pages-Xatart Pares, Xavier**
**B.P. 1751**
**Abidjan (CI)**
Inventeur : **Bouvron, Catherine (née Ferrenbach)**
**B.P. 1751**
**Abidjan (CI)**

(74) Mandataire : **Peuscet, Jacques**
**Cabinet Peuscet 3, Square de Maubeuge**
**F-75009 Paris (FR)**

## 0 062 565

### Procédé de fabrication d'esters de sucre et notamment de saccharose

La présente invention a trait à un procédé de fabrication des esters de sucre, et notamment de saccharose, et d'acides gras. Les esters gras de sucre ainsi obtenus sont utilisables notamment comme additifs pour les industries alimentaires, cosmétiques et pharmaceutiques.

On connaît déjà différents procédés de fabrication d'esters gras de sucre et notamment de saccharose. Ces procédés présentent souvent une certaine toxicité en raison des solvants, tels que la DMF qui sont utilisés.

Dans l'un de ces procédés, on effectue une estérification d'hydrates de carbone, et en particulier de sucre, en faisant réagir le sucre avec un anhydride d'acides gras, ou encore un mélange d'un anhydride d'acide, tel que, par exemple, un anhydride acétique, et d'un acide gras, la réaction d'estérification étant facilitée par l'adjonction d'un catalyseur. Un tel procédé ne permet cependant pas d'obtenir un rendement très élevé et est donc relativement coûteux.

Le procédé de préparation décrit dans FR-A-2 205 504 permet l'obtention d'un sucre acylé et estérifié par un acide gras avec une teneur en acide gras de l'ordre de 4 à 7 % en poids. La première étape du procédé est une acylation — notamment par l'acide acétique anhydre ou son anhydride — d'un hydrate de carbone pouvant être par exemple le saccharose ou le glucose. La seconde étape est une réaction d'estérification mettant en œuvre un acide gras ou son anhydride.

On connaît également déjà un procédé de préparation d'esters gras de sucre, et notamment de saccharose, dans lequel on fait réagir le sucre avec un alkyl-ester d'acide gras saturé ou insaturé, l'alkyl étant un alkyl à petit nombre de carbones, par exemple un méthyl. La réaction s'effectue à l'aide d'un catalyseur tel que le carbonate de potassium, l'ensemble étant ensuite repris dans l'acétone ou une autre cétone, une méthyle éthyle cétone, du chloroforme ou de l'acétone pour des opérations de filtration et de purification. Ce procédé ne permet qu'un rendement de l'ordre de 69 % exprimé en monostéarate de sucre et est donc aussi relativement coûteux.

La présente invention se propose de remédier à ces inconvénients et de fournir un procédé de fabrication d'esters gras de sucre, et notamment de saccharose, susceptible d'être mis en œuvre de façon simple, demandant une durée de préparation relativement réduite et aboutissant à un rendement par exemple exprimé en tristéarate de sucre acétylé supérieur à 80 %.

L'invention a donc pour objet un procédé de fabrication d'esters gras de sucre acylé et notamment d'esters gras de sucre acétylés, et plus particulièrement d'esters gras de saccharose acétylé, dans lequel, en premier lieu, on acyle le sucre par au moins un acide carboxylique en $C_2$-$C_4$ ou de préférence par l'anhydride correspondant et notamment par l'anhydride acétique, caractérisé par le fait que l'on extrait le sucre acylé obtenu et que l'on réalise ensuite, en présence d'un catalyseur, une interestérification du sucre acylé extrait avec un alkyl-ester d'acide gras dans lequel le groupe alkyl comporte un nombre d'atomes de carbone au plus égal à 4, notamment avec un méthylester d'acide gras. Les acides gras utilisés sont en général des acides gras à longues chaînes saturés ou insaturés ayant 8 à 22 atomes de carbone, de préférence, les acides palmitique ou stéarique ou un mélange de ces deux acides.

Selon un mode de mise en œuvre avantageux de l'invention, on préfère utiliser, à la place de l'acide carboxylique, l'anhydride correspondant. On préfère également l'anhydride en $C_2$ (anhydride acétique) à celui en $C_3$ (propionique), et ce dernier à celui en $C_4$ (butyrique).

De façon avantageuse, on effectue l'étape d'acylation en amenant du sucre, tel que du saccharose, de préférence finement broyé à l'état de sucre-glace, en solution dans de l'anhydride, de préférence, l'anhydride acétique en présence d'un catalyseur qui est un sel d'un acide organique faible, de préférence constitué par l'acétate de sodium. En général, la quantité de catalyseur utilisé est comprise entre 0,5 et 10 % en poids par rapport au sucre de départ, de préférence 5 %. On effectue l'étape d'acétylation en portant l'ensemble à la température d'ébullition sous reflux de l'anhydride, sous agitation et à la pression atmosphérique. L'acylation a lieu, de préférence, pendant une durée au moins égale à 3 et, de préférence, de l'ordre de 4 heures.

Après élimination, par exemple par distillation, de l'anhydride acétique en excès, le sucre acétylé est ensuite extrait à l'aide d'un solvant convenable, un alcool aliphatique partiellement soluble dans l'eau et capable de dissoudre largement les esters de sucre, de préférence le n-butanol, puis lavé à l'eau afin d'éliminer les traces d'acide acétique, et de catalyseur, après quoi le n-butanol est éliminé, par exemple par distillation.

Le rendement de l'étape d'acétylation dans le procédé selon l'invention est au moins de 80 % en poids exprimé en sucre acétylé par rapport au sucre de départ.

L'interestérification du sucre acylé avec un ou plusieurs esters d'acides gras s'effectue alors dans une réaction d'échange consistant à déplacer les molécules d'acide en $C_2$, $C_3$ ou $C_4$, notamment d'acide acétique, fixées sur le sucre et d'alcool méthylique fixées sur l'acide gras, de manière à fixer l'acide gras sur le sucre et à combiner l'acide carboxylique et l'alcool méthylique libéré pour former du carboxylate ou acétate de méthyle, cette réaction laissant, dans la pratique, sur l'ester obtenu, un ou plusieurs radicaux acylés substitués, de sorte que l'on obtient des sucres acylés interestérifiés constitués généralement de mono- et surtout de di- et/ou tri-esters d'acides gras acylés.

L'interestérification du sucre acylé avec le (ou les) ester(s) d'acides gras, notamment avec les esters méthyliques d'acides gras tels que les acides palmitique ou stéarique ou un mélange des deux et, en

**0 062 565**

rapport molaire compris entre 0,1 et 2 moles de sucre acylé pour 1 mole d'esters méthyliques, est effectuée, de préférence, en présence d'un catalyseur classique alcalin pour interestérification tel que du carbonate de potassium en quantité comprise entre 10 et 30 % en poids par rapport au sucre acylé et à une température de l'ordre de 140 °C sous pression atmosphérique pendant une durée au moins égale à 8 heures et, de préférence, de 8 à 10 heures. Pendant la réaction, le carboxylate de méthyle formé est, de préférence, recueilli. Le milieu réactionnel, à la fin de l'interestérification est ensuite amené à une température plus basse et, après élimination ou destruction du catalyseur, par exemple par addition d'acide acétique, les esters sont repris par un solvant convenable, par exemple de l'acétone ou un solvant halogéné ininflammable, de préférence le dichlorométhane ou le trichloro-trifluoroéthane ; l'ensemble est alors soumis à une filtration permettant de séparer le catalyseur détruit et le sucre acylé non réagi. Le solvant est ensuite éliminé et les sucres acylés et estérifiés sont récupérés : on agit, par exemple, par cristallisation dans un solvant approprié, tel que l'acétone, ou par distillation du solvant (notamment solvant halogéné).

Le rendement de l'étape d'interestérification conformément à l'invention est de l'ordre de 96 % par rapport au sucre acylé de départ et exprimé en tristéarate de sucre acylé.

D'autres avantages et caractéristiques de l'invention apparaîtront à la lecture de la description suivante faite à titre d'exemple non limitatif.

Exemple 1

On mélange trois kilogrammes de sucre ordinaire (saccharose) finement broyé dans 18 litres d'anhydride acétique avec adjonction de 150 grammes d'acétate de sodium ; on dissout le sucre dans l'anhydride acétique.

On porte cette solution à une température de 140 °C à la pression ambiante et on la maintient pendant une durée de 4 heures sous agitation à cette température, au reflux de l'anhydride acétique.

Le mélange est ensuite refroidi jusqu'à environ 55 °C et on procède alors à une distillation de l'anhydride acétique sous 30 à 45 mmHg permettant de récupérer 12,5 litres d'anhydride.

Le sucre acétylé ainsi obtenu subit ensuite une extraction au n-butanol suivie de trois lavages à l'eau et d'une décantation finale. L'extraction s'effectue dans 14 litres de n-butanol et on utilise pour chaque lavage 18 litres d'eau, de préférence à une température d'environ 60 °C.

On effectue ensuite une distillation sous vide partiel de 35 à 50 mmHg du n-butanol, à une température comprise entre 50 et 55 °C, ce qui permet d'obtenir le sucre acétylé désiré ainsi que du n-butanol récupéré.

Le rendement en sucre acétylé à cette étape est de 85 % par rapport au sucre de départ.

On effectue ensuite l'étape d'interestérification en chargeant dans un réacteur, puis en chauffant à 140 °C à pression ambiante, 3,5 kg de ce sucre acétylé, 7 kg d'un mélange d'ester méthylpalmitique et méthyl stéarique dans les proportions 1 : 1 et 1,1 kg de carbonate de potassium. Cette étape d'interestérification se poursuit pendant une durée de 8 heures environ. L'acétate de méthyle, qui se forme, est récupéré.

L'étape d'interestérification est poursuivie par une étape de refroidissement jusqu'à environ 80 °C, après quoi on ajoute 200 grammes d'acide acétique, pour détruire le carbonate de potassium, puis 13 litres d'acétone.

On effectue ensuite une filtration à chaud de l'ensemble permettant d'éliminer le catalyseur détruit et le sucre acétylé n'ayant pas réagi. Le filtrat est ensuite dilué dans 7 litres d'acétone et cristallisé à une température de 5 °C au maximum ; les cristaux de l'ester gras de sucre acétylé obtenu sont filtrés et recueillis ; le filtrat, contenant l'acétone, est éliminé. On obtient 6,5 kg de produit.

Le sucre acétylé et estérifié peut ensuite être broyé et conditionné pour son utilisation.

A titre d'exemple, trois lots d'esters palmito-stéarique de sucre acétylés selon l'invention, numérotés de 1 à 3, présentent les caractéristiques suivantes :

| Lot | Point de fusion | Indice d'acide | Indice de saponifi-cation | Indice d'hydroxyle | Indice d'iode |
|-----|-----------------|----------------|---------------------------|--------------------|---------------|
| N° 1 | 50 °C | 4,2 | 238 | 30 | 0,6 |
| N° 2 | 48,2 °C | 4,2 | 250 | 40 | 0,6 |
| N° 3 | 50,5 °C | 2,8 | 220 | 31 | 0,6 |

L'analyse calorimétrique différentielle révèle un thermogramme sans incident, confirmant la pureté du produit obtenu.

Exemple 2

On fabrique 3,5 kg de sucre acétylé selon le processus précédemment décrit. On effectue ensuite

3

l'étape d'interestérification en chargeant dans un réacteur puis en chauffant à 140 °C à pression ambiante, 3,5 kg de ce sucre acétylé, 7,34 kg d'ester méthylstéarique et 1,1 kg de carbonate de potassium. Cette étape d'interestérification se poursuit pendant 8 heures. L'acétate de méthyle qui se forme est récupéré.

L'étape d'interestérification est poursuivie par une étape de refroidissement jusqu'à environ 90 °C, après quoi on ajoute 200 g d'acide acétique pour détruire le carbonate de potassium puis 13 litres de méthyl éthyl cétone.

On effectue ensuite une filtration à chaud de l'ensemble. Le filtrat est ensuite dilué dans 7 litres de méthyl éthyl cétone et cristallisé à une température de 5 °C au maximum ; les cristaux de l'ester gras de sucre acétylé obtenu sont filtrés et recueillis. On obtient 6,83 kg.

Le sucre acétylé et estérifié peut ensuite être broyé et conditionné pour son utilisation.

A titre d'exemple, les caractéristiques d'un lot d'esters stéarique de sucre acétylés selon l'invention sont point de fusion 52 °C, indice d'acide 3,2, indice de saponification 215, indice d'hydroxyle 28, indice d'iode 0,4.

Exemple 3

On fabrique 3,5 kg de sucre acétylé selon le processus décrit dans l'exemple 1. On effectue ensuite l'étape d'interestérification en chargeant dans un réacteur puis en chauffant à 140 °C à pression ambiante, 3,5 kg de ce sucre acétylé, 7 kg d'un mélange d'ester méthyl palmitique et méthyl stéarique dans les proportions 1/1 et 1,1 kg de carbonate de potassium. Cette étape d'interestérification se poursuit pendant 8 heures au moins. L'acétate de méthyle, qui se forme, est récupéré.

L'étape d'interestérification est suivie d'une étape de refroidissement jusqu'à environ 60 °C après quoi on ajoute 200 g d'acide acétique pour détruire le carbonate de potassium puis 10 litres de dichlorométhane. Le milieu réactionnel est maintenu à 40 °C pendant 10 minutes. On effectue ensuite une filtration à chaud de l'ensemble, permettant d'éliminer le sucre acétylé, qui n'a pas réagi, et le catalyseur détruit. Le filtrat est replacé dans le réacteur et chauffé à température d'ébullition du solvant sous un léger vide de façon à évaporer et à recueillir ce dernier. On récupère au moins 8 l de solvant.

On obtient 7 kg de sucre acétylé estérifié sous forme d'une masse fondue de couleur jaune clair. Le sucre acétylé et estérifié peut ensuite être broyé et conditionné pour son utilisation.

A titre d'exemple, les caractéristiques d'un lot d'esters palmitique et stéarique de sucre acétylé selon l'invention sont : point de fusion : 42 °C ; indice d'acide : 5 ; indice de saponification : 220 ; indice d'hydroxyle : 35 ; indice d'iode : 0,5.

Bien que l'invention ait été décrite à propos d'un mode de mise en œuvre particulier de l'invention, il est bien entendu qu'elle n'y est nullement limitée et qu'on peut lui apporter diverses modifications de détail sans pour cela s'éloigner ni de son cadre, ni de son esprit.

**Revendications**

1. Procédé de fabrication d'esters gras de sucre acylé, dans lequel, en premier lieu, on acyle le sucre par au moins un acide carboxylique en $C_2$-$C_4$ ou par l'anhydride correspondant, caractérisé par le fait que l'on extrait le sucre acylé obtenu et que l'on réalise ensuite, en présence d'un catalyseur, une interestérification du sucre acylé extrait avec un alkyl-ester d'acide gras dans lequel le groupe alkyl comporte un nombre d'atomes de carbone au plus égal à 4.

2. Procédé de fabrication selon la revendication 1, caractérisé par le fait que l'on utilise pour l'acylation l'acide acétique, notamment sous la forme de l'anhydride acétique.

3. Procédé de fabrication selon l'une des revendications 1 et 2, caractérisé par le fait que le sucre utilisé est du saccharose.

4. Procédé de fabrication selon l'une des revendications 1 à 3, caractérisé par le fait que l'alkyl-ester d'acide gras utilisé est le méthyl-ester de l'acide gras.

5. Procédé de fabrication selon l'une des revendications 1 à 4, caractérisé par le fait que l'on effectue l'étape d'acylation à la température d'ébullition sous reflux de l'anhydride.

6. Procédé de fabrication selon la revendication 5, caractérisé par le fait que l'on effectue l'acylation sous agitation à la pression atmosphérique pendant une durée au moins égale à 3 à 4 heures.

7. Procédé de fabrication selon l'une des revendications 2 à 6, caractérisé par le fait que l'on effectue une extraction du sucre acylé à l'aide d'un solvant, notamment un alcool aliphatique partiellement soluble dans l'eau, notamment le n-butanol, suivie d'un lavage puis de l'élimination du solvant.

8. Procédé de fabrication selon l'une des revendications 3 à 6, caractérisé par le fait que l'on utilise, comme catalyseur pour l'acylation du sucre, un sel d'acide organique faible, notamment de l'acétate de sodium.

9. Procédé de fabrication selon l'une des revendications 1 à 8, caractérisé par le fait que l'on effectue l'étape d'interestérification à une température de l'ordre de 140 °C à la pression ambiante pendant une durée au moins égale à 8 heures.

10. Procédé de fabrication selon l'une des revendications 1 à 9, caractérisé par le fait que l'on réalise l'interestérification selon un rapport molaire compris entre 0,1 et 2 moles de sucre acylé pour un mole d'ester méthylique.

11. Procédé de fabrication selon l'une des revendications 1 à 10, caractérisé par le fait que l'on utilise comme catalyseur pour l'interestérification un catalyseur alcalin, notamment du carbonate de potassium.

12. Procédé de fabrication selon l'une des revendications 1 à 11, caractérisé par le fait que l'on détruit le catalyseur d'interestérification, que l'on reprend le produit de l'étape d'interestérification dans un solvant, que l'on effectue une filtration pour éliminer le catalyseur détruit et le sucre acylé n'ayant pas réagi.

13. Procédé de fabrication selon la revendication 12, caractérisé par le fait qu'après filtration pour éliminer le catalyseur et le sucre acylé n'ayant pas réagi, on effectue, pour recueillir l'ester gras de sucre, une cristallisation dans un solvant convenable, notamment l'acétone.

14. Procédé de fabrication selon la revendication 12, caractérisé par le fait qu'après filtration pour éliminer le catalyseur et le sucre acylé n'ayant pas réagi, on effectue une distillation du solvant utilisé, notamment un solvant halogéné tel que le dichlorométhane ou le trichloro trifluoroéthane, pour recueillir directement l'ester gras de sucre sous forme fondue.

15. Procédé de fabrication selon l'une des revendications 1 à 14, caractérisé par le fait que l'on utilise des acides gras à longues chaînes saturés ou insaturés ayant 8 à 22 atomes de carbone, notamment l'acide stéarique ou l'acide palmitique ou le mélange des deux.

## Claims

1. Process for the manufacture of fatty acid esters of acylated sugar, in which, firstly, the sugar is acylated with at least one $C_2$-$C_4$ carboxylic acid or with the corresponding anhydride, characterised in that the acylated sugar obtained is extracted and in that the extracted acylated sugar is then subjected to interesterification, in the presence of a catalyst, with a fatty acid alkyl ester in which the alkyl group contains at most 4 carbon atoms.

2. Manufacturing process according to Claim 1, characterised in that acetic acid, in particular in the form of acetic anhydride, is used for the acylation.

3. Manufacturing process according to one of Claims 1 and 2, characterised in that the sugar used is sucrose.

4. Manufacturing process according to one of Claims 1 to 3, characterised in that the fatty acid alkyl ester used is the fatty acid methyl ester.

5. Manufacturing process according to one of Claims 1 to 4, characterised in that the acylation step is carried out at the boiling point of the anhydride, under reflux.

6. Manufacturing process according to Claim 5, characterised in that the acylation is carried out with stirring, at atmospheric pressure, for a period of at least 3 to 4 hours.

7. Manufacturing process according to one of Claims 2 to 6, characterised in that the acylated sugar is extracted with a solvent, in particular a partially water-soluble aliphatic alcohol, especially n-butanol, this being followed by washing and then removal of the solvent.

8. Manufacturing process according to one of Claims 3 to 6, characterised in that a salt of a weak organic acid, in particular sodium acetate, is used as a catalyst for the acylation of the sugar.

9. Manufacturing process according to one of Claims 1 to 8, characterised in that the interesterification step is carried out at a temperature of the order of 140 °C, at ambient pressure, for a period of at least 8 hours.

10. Manufacturing process according to one of Claims 1 to 9, characterised in that the interesterification is carried out with a molar ratio of between 0.1 and 2 mols of acylated sugar per mol of methyl ester.

11. Manufacturing process according to one of Claims 1 to 10, characterised in that an alkaline catalyst, in particular potassium carbonate, is used as the catalyst for the interesterification.

12. Manufacturing process according to one of Claims 1 to 11, characterised in that the interesterification catalyst is destroyed, in that the product of the interesterification step is taken up in a solvent and in that filtration is carried out to remove the destroyed catalyst and the unreacted acylated sugar.

13. Manufacturing process according to Claim 12, characterised in that, after filtration to remove the catalyst and the unreacted acylated sugar, crystallisation is carried out from a suitable solvent, in particular acetone, in order to collect the fatty acid ester of the sugar.

14. Manufacturing process according to Claim 12, characterised in that, after filtration to remove the catalyst and the unreacted acylated sugar, the solvent used, in particular a halogenated solvent such as methylene chloride or trichlorotrifluoroethane, is distilled in order to collect the fatty acid ester of the sugar directly in the molten form.

15. Manufacturing process according to one of Claims 1 to 14, characterised in that saturated or unsaturated longchain fatty acids having 8 to 22 carbon atoms are used, in particular stearic acid, palmitic acid or a mixture of the two.

**Ansprüche**

1. Verfahren zur Herstellung von acylierten Zuckerfettsäureestern, bei dem man an erster Stelle den Zucker mit wenigstens einer $C_2$-$C_4$-Carbonsäure oder mit dem entsprechenden Anhydrid acyliert, dadurch gekennzeichnet, daß man den erhaltenen acylierten Zucker extrahiert unf man anschließend in Gegenwart eines Katalysators eine Interveresterung des extrahierten acylierten Zuckers mit einem Fettsäurealkylester, in dem der Alkylrest höchstens vier Kohlenstoffatome enthält, durchführt.

2. Herstellungsverfahren nach Anspruch 1, dadurch gekennzeichnet, daß man für die Acylierung Essigsäure, insbesondere in Form des Essigsäureanhydrids, verwendet.

3. Herstellungsverfahren nach einem der Ansprüche 1 und 2, dadurch gekennzeichnet, daß der verwendete Zucker Saccharose ist.

4. Herstellungsverfahren nach einem der Ansprüche 1 bis 3, dadurch gekennzeichnet, daß der verwendete Fettsäurealkylester der Methylester der Fettsäure ist.

5. Herstellungsverfahren nach einem der Ansprüche 1 bis 4, dadurch gekennzeichnet, daß man die Acylierungsstufe bei der Siedetemperatur des Anhydrids unter Rückfluß durchführt.

6. Herstellungsverfahren nach Anspruch 5, dadurch gekennzeichnet, daß man die Acylierung unter Rühren bei Normaldruck während einer Dauer von wenigstens 3 bis 4 Stunden durchführt.

7. Herstellungsverfahren nach einem der Ansprüche 2 bis 6, dadurch gekennzeichnet, daß man eine Extraktion des acylierten Zuckers mit Hilfe eines Lösungsmittels, insbesondere eines in Wasser teilweise löslichen Alkohols, insbesondere n-Butanol, mit anschließender Wäsche und dann die Entfernung des Lösungsmittels vornimmt.

8. Herstellungsverfahren nach einem der Ansprüche 3 bis 6, dadurch gekennzeichnet, daß man als Katalysator für die Acylierung des Zuckers ein Salz einer schwachen Säure, insbesondere Natriumacetat, verwendet.

9. Herstellungsverfahren nach einem der Ansprüche 1 bis 8, dadurch gekennzeichnet, daß man die Interveresterungsstufe bei einer Temperatur in der Grössenordnung von 140 °C bei Normaldruck während einer Dauer von wenigstens 8 Stunden durchführt.

10. Herstellungsverfahren nach einem der Ansprüche 1 bis 9, dadurch gekennzeichnet, daß man die Interveresterung bei einem Molverhältnis zwischen 0,1 und 2 Mol des acylierten Zuckers pro Mol Methylester durchführt.

11. Herstellungsverfahren nach einem der Ansprüche 1 bis 10, dadurch gekennzeichnet, daß man als Katalysator für die Interveresterung einen alkalischen Katalysator, insbesondere Kaliumcarbonat, verwendet.

12. Herstellungsverfahren nach einem der Ansprüche 1 bis 11, dadurch gekennzeichnet, daß man den Interveresterungskatalysator zerstört, das Produkt der Interveresterungsstufe in einem Lösungsmittel aufnimmt, und eine Filtration zur Entfernung des zerstörten Katalysators und des nicht umgesetzten acylierten Zuckers vornimmt.

13. Herstellungsverfahren nach Anspruch 12, dadurch gekennzeichnet, daß man nach der Filtration zur Entfernung des Katalysators und des nicht umgesetzten acylierten Zuckers zur Gewinnung des Zuckerfettsäureesters eine Kristallisation in einem geeigneten Lösungsmittel, insbesondere Aceton, durchführt.

14. Herstellungsverfahren nach Anspruch 12, dadurch gekennzeichnet, daß man nach der Filtration zur Entfernung des Katalysators und des nicht umgesetzten acylierten Zuckers eine Destillation des verwendeten Lösungsmittels, insbesondere eines halogenierten Lösungsmittels wie Dichlormethan oder Trichlortrifluorethan, durchführt, um den Zuckerfettsäureester direkt in geschmolzener Form zu gewinnen.

15. Herstellungsverfahren nach einem der Ansprüche 1 bis 14, dadurch gekennzeichnet, daß man gesättigte oder ungesättigte langkettige Fettsäuren mit 8 bis 22 Kohlenstoffatomen, insbesondere Stearinsäure oder Palmitinsäure oder das Gemisch der beiden, verwendet.